# EUROPEAN PATENT APPLICATION

(11) **EP 3 922 291 A1**
(43) Date of publication of application: **15.12.2021**
(21) Application number: 20752711.0
(22) Date of filing: 31.01.2020
(51) Int. Cl.: A61M 21/00, A61B 5/16, A61B 5/18

(54) **DEGREE OF WAKEFULNESS CONTROL DEVICE, DEGREE OF WAKEFULNESS CONTROL METHOD AND RECORDING MEDIUM**

(30) Priority: 04.02.2019 JP 2019018218
(71) Applicant: NEC Corporation, 108-8001 Tokyo (JP); Daikin Industries, Ltd., Osaka 530-8323 (JP)
(72) Inventor: KOGO Takuma, Tokyo 108-8001 (JP); NISHINO Atsushi, Osaka-shi, Osaka 530-8323 (JP); HASHIMOTO Satoshi, Osaka-shi, Osaka 530-8323 (JP)
(74) Representative: Vossius & Partner Patentanwälte Rechtsanwälte mbB
(86) International application number: PCT/JP2020/003688
(87) International publication number: WO 2020/162358

(57) **Abstract**

A arousal level control apparatus calculates, under a limitation related to a physical quantity that affects an arousal level of a subject, a setting value for controlling the arousal level of the subject by using a physical quantity prediction model for calculating the physical quantity based on a setting value of the physical quantity and an arousal level prediction model for calculating a prediction value of the arousal level based on the calculated physical quantity calculated, and sets the calculated setting value to a control target device that affects the physical quantity.

## Description

### TECHNICAL FIELD

The present invention relates to an arousal level control apparatus, an arousal level control method, and a recording medium.

### BACKGROUND ART

There has been proposed a technique in which a user's physiological information is acquired and the degree of user's wakefulness is calculated from the acquired physiological information (for example, Patent Documents 1, 2). Here, the arousal level is an index indicating the degree of wakefulness, and indicates that the lower the arousal level, the drowsier the subject is.

In the state where the arousal level is low, the work efficiency often decreases when the user performs a task, and it is therefore not an appropriate state for performing a task. Such a state tends to be undesirable for performing tasks because, for example, in office work, work efficiency decreases, and in driving a car, distracted driving increases.

Therefore, there have been proposed systems for controlling the environment of the user so that the arousal level is improved or brought into an appropriate range (Patent Documents 3, 4, and 5).

Patent Document 3 discloses a system for controlling the arousal level for a vehicle driver in which settings of environment control devices for air conditioning, lighting, and so forth are changed to predetermined settings when a prediction value of the arousal level of the user in the case where the current environmental state continues, falls below a predetermined threshold value.

Patent Document 4 discloses a system for controlling the arousal level for a vehicle driver in which intensities of devices that stimulate the five senses such as air conditioning and lighting and the combination are controlled on the basis of predetermined settings, according to where a user's current state is, in particular, how far away from the desired range a user's current state is in two-axis coordinates consisting of an evaluation axis representing drowsy-wakeful and an evaluation axis representing comfort-discomfort.

Patent Document 5 discloses a system for controlling the arousal level for a vehicle driver in which warm/cold stimuli caused by changes in temperature are given to the user by periodically switching the air conditioning device between predetermined operating modes (temperature, air volume setting), when the arousal level of the subject falls below a preset threshold value.

Moreover, there is a technique for acquiring and processing user information or information on their surrounding environment.

For example, the mood estimation system disclosed in Patent Document 6 is such that the mood of a subject is indexed based only on the heart rate of the subject, and if the index value deviates from a preliminarily set range, the mood of the subject is represented as an index value on the basis of the subject's multiple physiological information and multiple environmental information of the subject's surrounding environment.

Also, the air conditioning management system disclosed in Patent Document 7 calculates a predicted environmental value after a predetermined amount of time has elapsed on the basis of the environmental value detected by a detection device, and parameters of an air conditioning device are calculated on the basis of the environmental value and the predicted environmental value and are transmitted to the air conditioning device.

Moreover, in the arousal level maintenance method disclosed in Patent Document 8, the arousal level is detected from the deep body temperature of the worker such as tympanic temperature, and when the degree of the worker's wakefulness appears decreasing, the wakening effect of light stimulation is given to the worker by changing illuminance from an illuminance suitable for work to an even higher illuminance.

Furthermore, the drowsiness estimation device disclosed in Patent Document 9 includes a neural network having a structure with two layers, namely, an image processing neural network and a drowsiness estimation neural network. The image processing neural network estimates the age and gender of the user and also extracts specific behaviors and states of the user that indicate a state of drowsiness, such as closed eyes. The drowsiness estimation neural network obtains the drowsiness state of the user based on the result of extracting the specific behavior and the state of the user that indicate a state of drowsiness and on the detection result of an indoor environment information sensor while also taking the age and gender of the user into consideration.

Patent Document 9 discloses that the control unit of the air conditioning device calculates air-conditioning control content so that the estimated drowsiness level is equal to or less than a threshold value, and executes the calculated air-conditioning control.

Furthermore, Patent Document 9 discloses that if the desired change is not observed in the user's behavior and state, the estimated behavior in the drowsy state is potentially departing from that in the actual drowsy state, and therefore the estimation model is updated.

### [Prior Art Documents]

### [Patent Documents]

[Patent Document 1] Japanese Patent No. 6043933
[Patent Document 2] Japanese Unexamined Patent Application, First Publication No. 2018-134274
[Patent Document 3] Japanese Unexamined Patent Application, First Publication No. 2017-148604
[Patent Document 4] Japanese Unexamined Patent Application, First Publication No. 2018-025870
[Patent Document 5] Japanese Unexamined Patent Application, First Publication No. 2013-012029
[Patent Document 6] Japanese Unexamined Patent Application, First Publication No. 2018-088966
[Patent Document 7] Japanese Unexamined Patent Application, First Publication No. 2006-349288
[Patent Document 8] Japanese Unexamined Patent Application, First Publication No. H9-140799
[Patent Document 9] Japanese Patent No. 6387173

### SUMMARY OF THE INVENTION

### Problem to be Solved by the Invention

However, even with use of the devices disclosed in Patent Document 1 to Patent Document 9, it is still not possible to realize an indoor environment that maintains a mental and physical state with high work performance. Therefore, it is desirable to control the arousal level on the basis of prediction, in order to improve work performance of the subject.

The present invention has an object of providing an arousal level control apparatus, an arousal level control method, and a recording medium capable of solving the problems mentioned above.

### Means for Solving the Problem

According to a first example aspect of the present invention, a arousal level control apparatus includes:
a setting value calculation means for calculating, under a limitation related to a physical quantity that affects an arousal level of a subject, a setting value for controlling the arousal level of the subject by using a physical quantity prediction model for calculating the physical quantity based on a setting value of the physical quantity and an arousal level prediction model for calculating a prediction value of the arousal level based on the calculated physical quantity calculated; and
a setting means for setting the calculated setting value to a control target device that affects the physical quantity.

According to a second example aspect of the present invention, an arousal level control method includes:
calculating, under a limitation related to a physical quantity that affects an arousal level of a subject, a setting value for controlling the arousal level of the subject by using a physical quantity prediction model for calculating the physical quantity based on a setting value of the physical quantity and an arousal level prediction model for calculating a prediction value of the arousal level based on the calculated physical quantity calculated; and
setting the calculated setting value to a control target device that affects the physical quantity.

According to a third example aspect of the present invention, a recording medium stores a program which causes a computer to execute steps of:
calculating, under a limitation related to a physical quantity that affects an arousal level of a subject, a setting value for controlling the arousal level of the subject by using a physical quantity prediction model for calculating the physical quantity based on a setting value of the physical quantity and an arousal level prediction model for calculating a prediction value of the arousal level based on the calculated physical quantity calculated; and
setting the calculated setting value to a control target device that affects the physical quantity.

### Effect of the Invention

According to the present invention, it is possible to realize an indoor environment that maintains a mental and physical state for high work performance.

### BRIEF DESCRIPTION OF THE DRAWINGS

FIG. 1 is a schematic block diagram showing an example of a device configuration of an arousal level control system according to an example embodiment.
FIG. 2 is a schematic block diagram showing an example of a functional configuration of an arousal level control apparatus according to an example embodiment.
FIG. 3 is a flowchart showing a first example of a processing procedure in which the setting value calculation unit according to the example embodiment calculates a device setting value and sets it in an environment control device.
FIG. 4 is a flowchart showing a second example of the processing procedure in which the setting value calculation unit according to the example embodiment calculates a device setting value and sets it in the environment control device.
FIG. 5 is a diagram showing an example of a configuration of the arousal level control apparatus according to the example embodiment.

### EXAMPLE EMBODIMENT

Hereinafter, example embodiments of the present invention are described; however, the present invention within the scope of the claims is not limited by the following example embodiments. Furthermore, not all the combinations of features described in the example embodiment may not be essential for the solving means of the invention.

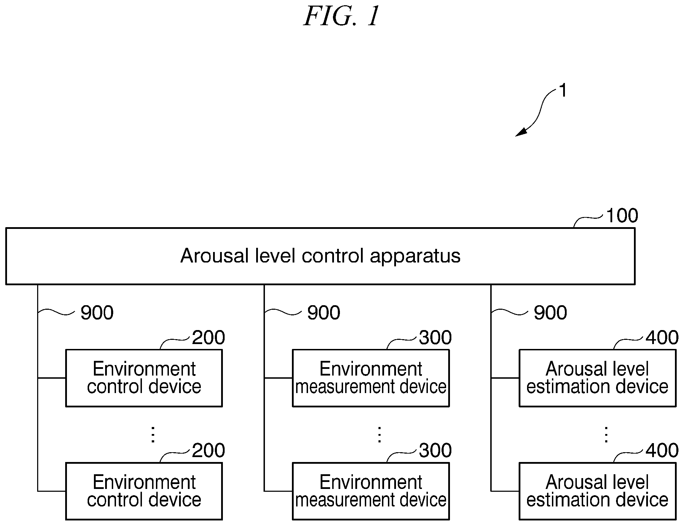

FIG. 1 is a schematic block diagram showing an example of a device configuration of an arousal level control system 1 according to an example embodiment. In the configuration shown in FIG. 1, the arousal level control system 1 includes an arousal level control apparatus 100, one or more environment control devices 200, one or more environment measurement devices 300, and one or more arousal level estimation devices 400.

The arousal level control apparatus 100 is connected to each of the environment control devices 200, the environment measurement devices 300, and the arousal level estimation devices 400 via a communication line 900 respectively, and can communicate with these devices. The communication line 900 may be configured with any of a dedicated line, the Internet, a VPN (Virtual Private Network), a LAN (Local Area Network) regardless of the access method of the communication line or the physical form of the communication line such as a wired line or a wireless line.

The arousal level control system 1 determines the arousal level of the subject of arousal level control, and controls the physical quantity of the surrounding environment where the subject of the arousal level control is present according to the determination result, to maintain or improve the arousal level. As mentioned above, the arousal level is an index indicating the degree of wakefulness, and it indicates that the lower the arousal level, the drowsier the subject of arousal level control is.

The subject of arousal level control may also be referred to as a user or simply as a subject.

Here, the physical quantity of the surrounding environment where the subject is present is a physical quantity (a quantity in a physical sense) that affects a subject, and, in this case, is a physical quantity that affects the arousal level of the subject in particular. The physical quantity of the surrounding environment where the subject is present is also simply referred to as a physical quantity.

Examples of physical quantities include, but are not limited to, ambient temperature such as room temperature and brightness such as illuminance of lighting equipment. For example, the arousal level control system 1 may give the subject stimuli other than temperature and brightness, such as humidity (dampness), sound or vibration, in addition to or instead of temperature and brightness, and may use the degree of these stimuli as physical quantities.

Hereinafter, the ambient temperature is simply referred to as temperature. However, the arousal level control system 1 may control other temperatures in addition to or instead of ambient temperature. For example, a heater may be provided on the seat surface of the subject's seat, and the arousal level control system 1 may control the temperature of the heater. That is to say, the arousal level control system 1 may control the temperature of something that comes in direct contact with the subject.

The unit by which the arousal level control system 1 controls physical quantities is not limited to a specific one. For example, a spot-type air conditioning device (a local air conditioner) and a lighting stand may be installed at the seat of a person, and the arousal level control system 1 may control physical quantities on a seat-by-seat basis. Alternatively, the arousal level control system 1 may control physical quantities on a room-by-room basis, or may control physical quantities of an entire building. Moreover, when controlling the physical quantities of an entire building, subjects need not be everyone in the building, but may be some of them in the building.

The number of subjects may be one or may be more than one. Only specific persons may become subjects by the arousal level control system 1 accepting registration of subjects. Alternatively, unspecified persons located in a control target space of the arousal level control system 1 may become subjects. In a case where there are a plurality of subjects, the arousal level control system 1 may control physical quantities for each subject, or may commonly control physical quantities of the plurality of subjects.

In order to improve the arousal level of a subject, it is conceivable to control a physical quantity so as to decrease the comfort, depending on the person, by raising the room temperature or brightening the lighting for example. The arousal level control system 1 determines the arousal level of the arousal level control subject and controls physical quantities according to the determination result, so that it is possible to achieve a balance between ensuring the arousal level and the comfort of the subject. For example, the arousal level control system 1 may control physical quantities so that the arousal level is improved only when the arousal level of the subject decreases.

In the following description, a case where the arousal level control system 1 improves the arousal level (shakes off drowsiness) of a subject is taken as an example; however, the arousal level control system 1 may decrease the arousal level of (induce sleep in) a subject. For example, the arousal level control system 1 may switch between executing control for improving the arousal level and executing control for decreasing the arousal level, depending on the time period. Alternatively, in a case where the arousal level of a subject is predicted to decrease, the arousal level control system 1 may execute control so that the arousal level of the subject does not decrease (that is to say, the subject does not become drowsy). Or, in a case where the arousal level of a subject is predicted to improve, the arousal level control system 1 may execute control so that the arousal level of the subject does not improve (that is to say, the subject does not awaken).

The arousal level control apparatus 100 controls the environment control device 200 according to the arousal level of the subject. The arousal level control apparatus 100 controls the physical quantity of the surrounding environment of the subject by controlling the environment control device 200, thereby controlling the arousal level of the subject.

The arousal level control apparatus 100 is configured, using a computer such as a personal computer (PC) or a workstation, for example.

The environment control device 200 is a device that adjusts physical quantities. As mentioned above, examples of physical quantities include ambient temperature and illuminance. Temperature can be adjusted by an air conditioning device, and illuminance can be adjusted by a lighting device. As described above, examples of the environment control device 200 include, but are not limited to, an air conditioning device and a lighting device.

The environment control device 200 corresponds to an example of a control target device, and is controlled by the arousal level control apparatus 100 as mentioned above.

A device other than the environment control device 200, such as the arousal level control apparatus 100, can acquire information on the operating state such as a device setting value from the environment control device 200, and can update the device setting value for the environment control device 200. Here, the device setting value is a physical quantity set in the environment control device 200 as a control target value. The device setting value may also be referred to as a physical quantity setting value or simply a setting value.

When the environment control device 200 is an air conditioning device, a set temperature can be used as a device setting value. When the environment control device 200 is a lighting device, a lighting output (such as light intensity, illuminance, electric current value, and electric power value) can be used as a device setting value. In the following description, a case where illuminance is used as a device setting value of the lighting device is taken as an example, but it is not limited to such an example.

The environment measurement device 300 is a device that measures physical quantities such as temperature and illuminance and converts them into numerical data. Examples of the environment measurement device 300 include, but are not limited to, a temperature sensor and an illuminance sensor.

The arousal level estimation device 400 is a device that estimates the arousal level of a subject from physiological information or the like and converts it into numerical data. The arousal level estimation device 400 may use any one or a combination of body temperature, video of a face, and pulse wave as physiological information; however, it is not limited to this example. The arousal level estimation device 400 measures or calculates physiological information, and converts the obtained physiological information into a numerical value (arousal level) indicating the arousal level.

The arousal level estimation device 400 is not essential for the arousal level control system 1. When the arousal level control system 1 does not include the arousal level estimation device 400, the arousal level of the subject is estimated on the basis of a physical quantity.

Next, a functional configuration of the arousal level control apparatus 100 will be described.

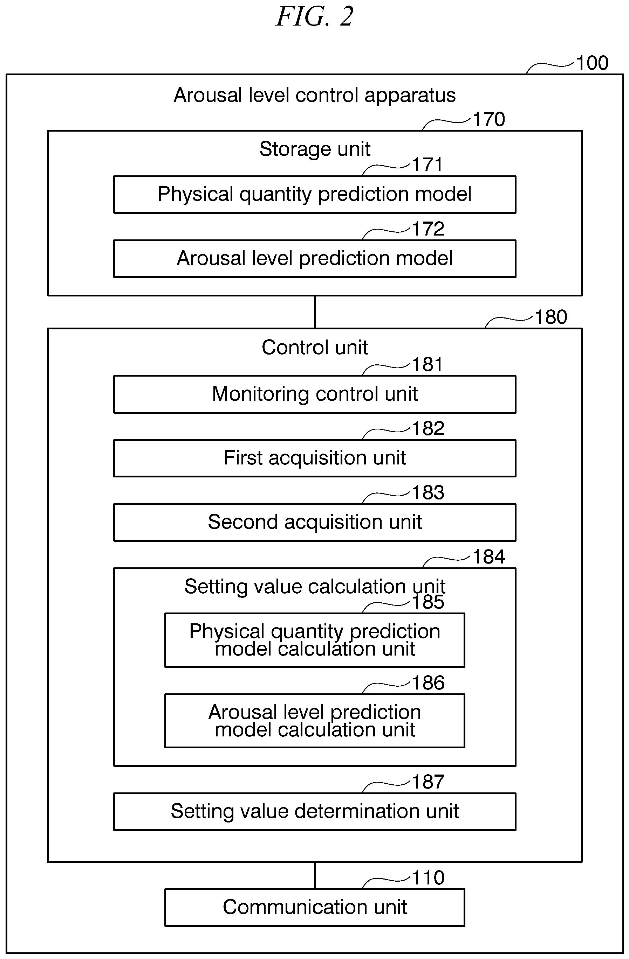

FIG. 2 is a schematic block diagram showing an example of a functional configuration of the arousal level control apparatus 100. In the configuration shown in FIG. 2, the arousal level control apparatus 100 includes a communication unit 110, a storage unit 170, and a control unit 180. The storage unit 170 includes a physical quantity prediction model 171 and an arousal level prediction model 172. The control unit 180 includes a monitoring control unit 181, a first acquisition unit 182, a second acquisition unit 183, a setting value calculation unit 184, a physical quantity prediction model calculation unit 185, an arousal level prediction model calculation unit 186, and a setting value determination unit 187.

The communication unit 110 communicates with other devices according to the control of the control unit 180. In particular, the communication unit 110 receives various information from each of the environment control devices 200, the environment measurement devices 300, and the arousal level estimation devices 400. Moreover, the communication unit 110 transmits device setting values to the environment control devices 200.

The storage unit 170 stores various types of information. The storage unit 170 is configured using a storage device included in the arousal level control apparatus 100.

The physical quantity prediction model 171 is a model for calculating a prediction value of a physical quantity on the basis of a setting value of the physical quantity (device setting value).

More specifically, the physical quantity prediction model 171, on the basis of the measurement value of the physical quantity measured by the environment measurement device 300 and the setting value of the physical quantity set in the environment control device 200, calculates a prediction value of the physical quantity at a time at which a predetermined amount of time has elapsed.

The time at which the predetermined amount of time has elapsed in this case is the time at which the predetermined amount of time has elapsed since the moment at which the physical quantity given to the physical quantity prediction model 171 was measured. Instead of the measurement time of the physical quantity given to the physical quantity prediction model 171, the time at which the arousal level control apparatus 100 (communication unit 110) has received the physical quantity can be used.

The predetermined amount of time in this case may be a certain fixed amount of time or may be variable as a model parameter. The model parameter referred to here is a setting parameter of the physical quantity prediction model 171. The value of a model parameter is referred to as model parameter value.

The arousal level prediction model 172 is a model for calculating a prediction value of an arousal level on the basis of a prediction value of the physical quantity calculated by the physical quantity prediction model 171. Furthermore, the arousal level prediction model 172 calculates a prediction value of an arousal level on the basis of a variation in a physical quantity in addition to a prediction value of the physical quantity. More specifically, the arousal level prediction model 172 calculates, on the basis of physical quantities, the prediction value of variation in the arousal level of a subject at a moment at which a predetermined amount of time has elapsed.

The control unit 180 controls each unit of the arousal level control apparatus 100 and executes various processes. The control unit 180 is realized by a CPU (Central Processing Unit) included in the arousal level control apparatus 100 reading out a program from the storage unit 170 and executing the program.

The monitoring control unit 181 communicates with the environment control device 200 via the communication unit 110. In communication with the environment control device 200, the monitoring control unit 181 acquires a device setting value set in the environment control device 200. Moreover, the monitoring control unit 181 updates the device setting value of the environment control device 200 by communicating with the environment control device 200. For example, the monitoring control unit 181 communicates with the environment control device 200 at constant intervals, and saves the device setting value acquired through the communication, together with the timestamp of the time of acquisition thereof (of the time of reception thereof). The term "save" here means causing the storage unit 170 to memorize a device setting value and timestamp therein.

In this manner, the monitoring control unit 181 sets a device setting value in the control target device. The monitoring control unit 181 corresponds to an example of a setting unit.

The monitoring control unit 181 sets a device setting value, a device setting value calculated by the setting value calculation unit 184, or a device setting value determined by the setting value determination unit 187, in the environment control device 200.

The first acquisition unit 182 communicates with the environment measurement device 300 via the communication unit 110, and acquires the measurement value of the physical quantity measured by the environment measurement device 300. For example, the first acquisition unit 182 communicates with the environment measurement device 300 at constant intervals, and saves the measurement value of the physical quantity acquired through the communication, together with the timestamp of the time of acquisition thereof (of the time of reception thereof). This timestamp can be interpreted as indicating the time at which the physical quantity is measured by the environment measurement device 300.

The second acquisition unit 183 communicates with the arousal level estimation device 400 and acquires an estimated value of the arousal level of the subject. For example, the second acquisition unit 183 communicates with the arousal level estimation device 400 at constant intervals, and saves the estimated value of the arousal level acquired through the communication, together with the timestamp of the time of acquisition thereof (of the time of reception thereof). This timestamp can be interpreted as indicating the time at which the estimation of the arousal level is made by the arousal level estimation device 400.

The estimated value of the arousal level of the subject is also referred to as the arousal level estimated value.

The setting value calculation unit 184 calculates a device setting value of the environment control device 200 so as to improve the arousal level of a user. For example, the setting value calculation unit 184 calculates a device setting value at constant intervals. The setting value calculation unit 184 acquires a device setting value from the monitoring control unit 181, acquires a measurement value of a physical quantity from the first acquisition unit 182, acquires an arousal level estimated value from the second acquisition unit 183, and calculates a device setting value on the basis of these acquired values. The setting value calculation unit 184 outputs the calculated device setting value to the monitoring control unit 181. The monitoring control unit 181 transmits the device setting value acquired from the setting value calculation unit 184 to the environment control device 200 via the communication unit 110, to thereby set it in the environment control device 200.

The setting value calculation unit 184 calculates a device setting value so that the arousal level becomes even higher.

The physical quantity prediction model calculation unit 185 reads out the physical quantity prediction model 171 from the storage unit 170 and executes it. Therefore, the physical quantity prediction model calculation unit 185 executes physical quantity prediction using the physical quantity prediction model 171.

The arousal level prediction model calculation unit 186 reads out the arousal level prediction model 172 from the storage unit 170 and executes it. Therefore, the arousal level prediction model calculation unit 186 executes the prediction of an arousal level using the arousal level prediction model 172.

The arousal level prediction model calculation unit 186 acquires, as learning data, a measurement value of the physical quantity from the first acquisition unit 182, and acquires, as learning data, an arousal level estimated value from the second acquisition unit 183.

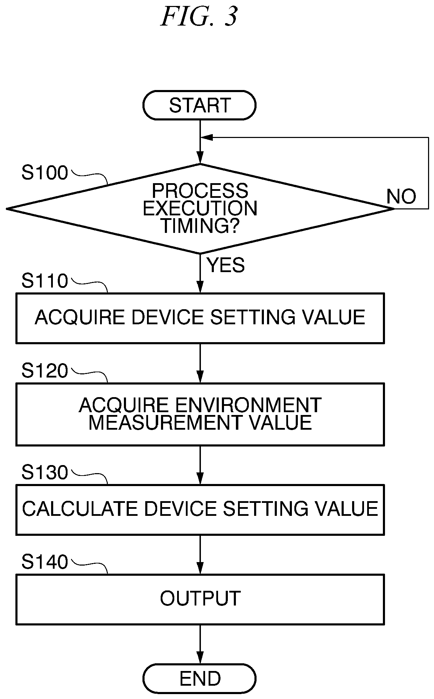

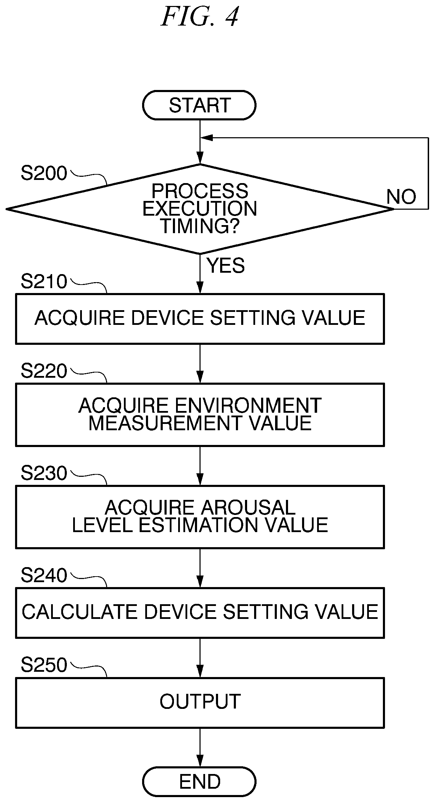

The setting value calculation unit 184 executes calculation through the procedure shown in FIG. 3 or FIG. 4. It is preferable that the calculation be executed at constant intervals of Δτ.

FIG. 3 is a flowchart showing a first example of a processing procedure in which the setting value calculation unit 184 calculates a device setting value and sets it in the environment control device 200. FIG. 3 shows an example of a case in which the setting value calculation unit 184 calculates a device setting value without using an arousal level estimated value.

In the process of FIG. 3, the setting value calculation unit 184 determines whether or not the execution timing of the process for calculating a device setting value has arrived (Step S100). If the execution timing is determined as having not arrived (Step S100: No), the process returns to Step S100. As a result, the setting value calculation unit 184 waits for the execution timing of the process for calculating a device setting value to arrive.

On the other hand, if the execution timing of the process for calculating a device setting value is determined as having arrived (Step S100: Yes), the setting value calculation unit 184 acquires a device setting value from the monitoring control unit 181 (Step S110).

The setting value calculation unit 184 acquires an environment measurement value (measurement value of the physical quantity measured by the environment measurement device 300) from the first acquisition unit 182 (Step S120). Then, the setting value calculation unit 184 calculates a device setting value (value for updating device setting value) (Step S130). In Step S130, the setting value calculation unit 184 calculates the device setting value without using an arousal level estimated value.

The setting value calculation unit 184 outputs the obtained device setting value to the monitoring control unit 181 (Step S140). The monitoring control unit 181 transmits the device setting value obtained from the setting value calculation unit 184 to the environment control device 200 via the communication unit 110, to thereby set the device setting value in the environment control device 200.

After Step S140, the setting value calculation unit 184 ends the process of FIG. 3.

FIG. 4 is a flowchart showing a second example of a processing procedure in which the setting value calculation unit 184 calculates a device setting value and sets it in the environment control device 200. FIG. 4 shows an example of a case in which the setting value calculation unit 184 calculates a device setting value, using an arousal level estimated value.

Step S200 to Step S220 of FIG. 4 are similar to Step S100 to Step S120 of FIG. 3.

After Step S220, the setting value calculation unit 184 acquires an arousal level estimated value from the setting value calculation unit 183 (Step S230).

Then, the setting value calculation unit 184 calculates a device setting value (value for updating device setting value) (Step S240). In Step S240, the setting value calculation unit 184 calculates the device setting value, using the arousal level estimated value.

Step S250 is similar to Step S140 of FIG. 3.

After Step S250, the setting value calculation unit 184 ends the process of FIG. 4.

As described above, the physical quantity prediction model 171 calculates a physical quantity that affects the arousal level of the subject. The arousal level prediction model 172 calculates a prediction value of an arousal level on the basis of the physical quantity calculated by the physical quantity prediction model 171. The setting value calculation unit 184 uses the physical quantity prediction model 171 and the arousal level prediction model 172 to calculate a setting value (device setting value) for controlling the arousal level of the subject, under limitations related to the physical quantity. The monitoring control unit 181 sets the calculated setting value in the environment control device 200.

In this way, the physical quantity prediction model 171 calculates the prediction value of the physical quantity, and the arousal level prediction model 172 predicts the arousal level using the prediction value of the physical quantity. Thereby, the physical quantity can be incorporated into the prediction of arousal level. According to the arousal level control apparatus 100, in this respect, when performing arousal level control, it is possible to more accurately presume the effect of its work on the surrounding environment on the arousal level.

Also, the setting value calculation unit 184 calculates setting values so that the arousal level becomes even higher.

According to the arousal level control apparatus 100, it is possible to improve the arousal level of the subject, and it is possible, for example, to improve the work efficiency when the subject is performing tasks.

The sum value or the mean value of prediction values of the variation in the arousal level is calculated for one or more subjects.

Also, the setting value calculation unit 184 calculates a setting value that satisfies the limitations related to comfort scores calculated for setting values.

As a result, the setting value calculation unit 184 can calculate a setting value in consideration of not only arousal level but also comfort. In this respect, the arousal level control apparatus 100 can balance arousal level and comfort.

Furthermore, the arousal level prediction model 172 calculates a prediction value of an arousal level on the basis of a physical quantity.

It is conceivable that the arousal level responds sensitively to the magnitude of physical quantities, and with the arousal level prediction model 172 predicting arousal level on the basis of the magnitude of the physical quantity, highly accurate prediction of arousal level is expected to be possible.

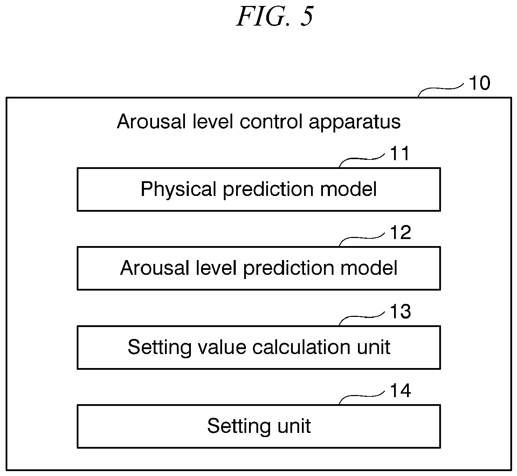

Next, the configuration of the example embodiment of the present invention will be described, with reference to FIG. 5.

FIG. 5 is a diagram showing an example of a configuration of an arousal level control apparatus 10 according to the example embodiment. The arousal level control apparatus 10 shown in FIG. 5 includes a physical quantity prediction model 11, an arousal level prediction model 12, a setting value calculation unit 13, and a setting unit 14.

In this configuration, the physical quantity prediction model 11 calculates a prediction value of a physical quantity on the basis of a setting value of the physical quantity that affects the arousal level of the subject. The arousal level prediction model 12 calculates a prediction value of the arousal level on the basis of the prediction value. The setting value calculation unit 13 uses the physical quantity prediction model and the arousal level prediction model to calculate the setting value for controlling the arousal level of the subject, under limitations related to physical quantity. The setting unit 14 sets the calculated setting value to a control target device that affects the physical quantity.

In this way, the physical quantity prediction model 11 calculates the prediction value of the physical quantity and the arousal level prediction model 12 predicts the arousal level using the prediction value of the physical quantity. As a result, the variation in physical quantity can be incorporated into the prediction of arousal level. According to the arousal level control apparatus 10, in this respect, when performing arousal level control, it is possible to more accurately presume the effect of its work on the surrounding environment on the arousal level.

The configuration of the arousal level control apparatus 100 is not limited to a configuration using a computer. For example, the arousal level control apparatus 100 may be configured, using dedicated hardware such as an ASIC (Application Specific Integrated Circuit).

In the present invention, arbitrary processes can also be realized by causing a CPU (Central Processing Unit) to execute a computer program.

In such a case, the program can be stored using various types of non-transitory computer readable media to be supplied to a computer. Non-transitory computer-readable media include various types of tangible storage media. Examples of non-transitory computer-readable media include a magnetic recording medium (such as a flexible disk, a magnetic tape, or a hard disk drive), a magnetic optical recording medium (such as a magnetic optical disk), a CD-ROM (Read Only Memory), a CD-R, a CD-R/W, a DVD (Digital Versatile Disc), a BD (Blu-ray (registered trademark) Disc), and a semiconductor memory (such as a mask ROM, a PROM (Programmable ROM), an EPROM (Erasable PROM), a flash ROM, or a RAM (Random Access Memory)).

This application is based upon and claims the benefit of priority from Japanese patent application No. 2019-018218, filed February 4, 2019, the disclosure of which is incorporated herein in its entirety by reference.

### Reference Symbols

- 1: Arousal level control system
- 10, 100: Arousal level control apparatus
- 11, 171: Physical quantity prediction model
- 12, 172: Arousal level prediction model
- 13, 184: Setting value calculation unit (setting value calculation means)
- 14: Setting unit (setting means)
- 110: Communication unit (communication means)
- 170: Storage unit (storage means)
- 180: Control unit (control means)
- 181: Monitoring control unit (monitoring control means)
- 182: First acquisition unit (first acquisition means)
- 183: Second acquisition unit (second acquisition means)
- 185: Physical quantity prediction model calculation unit (physical quantity prediction model calculation means)
- 186: Arousal level prediction model calculation unit (arousal level prediction model calculation means)
- 187: Setting value determination unit (setting value determination means)
- 200: Environment control device
- 300: Environment measurement device
- 400: Arousal level estimation device

## Claims

1. A arousal level control apparatus comprising:
a setting value calculation means for calculating, under a limitation related to a physical quantity that affects an arousal level of a subject, a setting value for controlling the arousal level of the subject by using a physical quantity prediction model for calculating the physical quantity based on a setting value of the physical quantity and an arousal level prediction model for calculating a prediction value of the arousal level based on the calculated physical quantity calculated; and
a setting means for setting the calculated setting value to a control target device that affects the physical quantity.

2. The arousal level control apparatus according to claim 1, wherein the setting value calculation means calculates the setting value so that the arousal level becomes even higher.

3. The arousal level control apparatus according to claim 2, wherein the setting value calculation means calculates the setting value so that a sum or a mean value of prediction values of a variation in the arousal level becomes even greater for the one subject or more subjects.

4. The arousal level control apparatus according to any one of claims 1 to 3, wherein the setting value calculation means calculates the setting value that satisfies a limitation related to comfort scores calculated for the setting value.

5. An arousal level control method comprising:
calculating, under a limitation related to a physical quantity that affects an arousal level of a subject, a setting value for controlling the arousal level of the subject by using a physical quantity prediction model for calculating the physical quantity based on a setting value of the physical quantity and an arousal level prediction model for calculating a prediction value of the arousal level based on the calculated physical quantity calculated; and
setting the calculated setting value to a control target device that affects the physical quantity.

6. A recording medium storing a program which causes a computer to execute steps of:
calculating, under a limitation related to a physical quantity that affects an arousal level of a subject, a setting value for controlling the arousal level of the subject by using a physical quantity prediction model for calculating the physical quantity based on a setting value of the physical quantity and an arousal level prediction model for calculating a prediction value of the arousal level based on the calculated physical quantity calculated; and
setting the calculated setting value to a control target device that affects the physical quantity.
